Europäisches Patentamt

European Patent Office

Office européen des brevets

(19)

(11) Numéro de publication : **0 336 827 B1**

(12) **FASCICULE DE BREVET EUROPEEN**

(45) Date de publication du fascicule du brevet :
**22.07.92 Bulletin 92/30**

(51) Int. Cl.⁵ : **G01N 33/94,** G01N 33/52

(21) Numéro de dépôt : **89400907.5**

(22) Date de dépôt : **03.04.89**

(54) **Procédé de détection de traces de stupéfiants et produits pour la mise en oeuvre de ce procédé.**

(30) Priorité : **08.04.88 FR 8804677**

(43) Date de publication de la demande :
**11.10.89 Bulletin 89/41**

(45) Mention de la délivrance du brevet :
**22.07.92 Bulletin 92/30**

(84) Etats contractants désignés :
**AT BE CH DE ES GB GR IT LI LU NL**

(56) Documents cités :
**EP-A- 0 229 517
EP-A- 0 233 063
DE-A- 2 206 697
US-A- 3 912 655**

(73) Titulaire : **Le Fel, Marie France
1, Place du Domaine de la Peyrière
F-06250 Mougins (FR)**
Titulaire : **Cordell, Christopher
1, Place du Domaine de la Peyrière
F-06250 Mougins (FR)**

(72) Inventeur : **Le Fel, Marie France
1, Place du Domaine de la Peyrière
F-06250 Mougins (FR)**
Inventeur : **Cordell, Christopher
1, Place du Domaine de la Peyrière
F-06250 Mougins (FR)**

(74) Mandataire : **Loyer, Bertrand et al
Cabinet Pierre Loyer 77, rue Boissière
F-75116 Paris (FR)**

EP 0 336 827 B1

## Description

La présente invention a pour objet un procédé de détection de traces de stupéfiants tels que l'héroïne, la cocaïne et le cannabis.

Actuellement pour vérifier si un produit est un stupéfiant, on procède à une analyse chimique complète sur un échantillon. Non seulement une telle méthode est longue, mais encore elle ne peut être mise en oeuvre que si l'on dispose d'un échantillon important. La présente méthode permet de détecter des traces infinitésimales des drogues susnommées par projection d'un réactif coloré sur toute surface ayant été en contact avec un de ces stupéfiants. Par exemple, en pulvérisant le réactif sur une surface ayant été touchée par quelqu'un ayant manipulé de la drogue, on peut détecter la présence de particules infinitésimales de drogue. Ou encore en frottant avec un papier absorbant blanc, genre KLEENEX un vêtement d'une personne ayant été en contact avec de la drogue on peut recueillir sur ce papier des particules infinitésimales de drogue qui seront colorées par la projection du réactif.

De même si de la drogue est mélangée à un parfum par exemple, on verse quelques gouttes de ce parfum sur un papier absorbant et on pulvérise le réactif sur ce papier : le réactif changera de couleur au contact des particules de drogue dissoutes dans le parfum ayant imprégné le papier.

Le procédé selon l'invention consiste donc à pulvériser un réactif coloré sur un support sur lequel se trouvent des particules de drogue, même en quantités infinitésimales.

La présente invention concerne également des produits pour la mise en oeuvre de ce procédé.

Le premier est destiné à la détection de la cocaïne : il agit par réaction sur les alcaloïdes spécifiques de la cocaïne ou de ses dérivés. Ce produit est constitué par une matière active comportant un mélange de Chlorure de Cobalt et Thiocyanate d'ammonium, cette matière active étant fortement diluée dans au moins 5Ø% d'eau.

Il s'avère en effet que si la concentration est trop forte, le risque d'obtenir des résultats positifs (changement de couleur) alors qu'il n'y a pas de particules de cocaïne est très important. Il faut donc une solution très diluée. Plus on dilue la solution, plus on élimine les risques de "faux positifs", mais si la dilution est trop forte, on risque de ne plus rien détecter.

Les recherches ont montré que les résultats les meilleurs étaient obtenus avec la composition suivante:

Chlorure de cobalt           1%
Thiocyanate d'ammonium   3%
Eau                                  96%

On peut augmenter la dilution jusqu'à vingt fois et on a alors :

Chlorure de cobalt           Ø,Ø5%
Thiocyanate d'ammonium  Ø,15%
Eau                                  99,8Ø%

ce qui fait une proportion de 2 pour mille de matières actives par rapport à l'eau.

Le liquide ainsi obtenu est mis dans un pulvérisateur qui peut être soit un pulvérisateur à pompe, soit un pulvérisateur par aérosol. Dans ce dernier cas, pour éviter une congélation du produit à la buse de pulvérisation, on peut substituer de l'éthanol à l'eau jusque dans une proportion de 1Ø%.

Comme indiqué précédemment, les deux constituants de la matière active sont présents dans la proportion de 25% pour le Chorure de Cobalt et de 75% pour le Thiocyanate d'ammonium. Cette proportion est préférentielle ; cependant, elle peut varier de 1Ø% à 75% pour le Chlorure de Cobalt et donc de façon correspondante de 9Ø% à 25% pour le Thiocyanate d'ammonium.

Le produit est incolore : lorsqu'il réagit sur les alcaloïdes spécifiques de la cocaïne, il vire au bleu turquoise.

Le deuxième produit selon l'invention est destiné à la détection de l'héroïne. Il est constitué par une solution dans l'eau, éventuellement additionnée d'éthanol, de Bromophénol bleu.

De préférence, la solution contient 4% de Bromophénol bleu pour 96% d'eau ; ces proportions peuvent varier entre 1% de Bromophénol bleu pour 99% d'eau et 75% pour 25% d'eau.

Le Bromophénol liquide réagit sur l'acide acétique ayant servi à la transformation de la morphine base en héroïne et se colore en noir du fait de cette réaction.

Dans ce cas, comme dans le cas de la détection de la cocaïne, une trop forte concentration en matière active risque de donner des "faux positifs", c'est-à-dire une indication de présence d'héroïne, alors qu'il n'y en a pas : il est donc préférable d'utiliser des concentrations faibles (4% de Bromophénol bleu pour 96% d'eau). Cependant, dans ce cas également, il y a une limite inférieure à ne pas dépasser (environ 1%) sans cela la réaction est trop faible et n'est plus visible.

Comme précédemment, le liquide est pulvérisé au moyen d'un pulvérisateur à pompe ou à aérosols et dans ce cas également, on peut remplacer une partie de l'eau par de l'éthanol et cela jusqu'à environ 2Ø%.

Le produit est légèrement coloré en bleu : lorsqu'il rencontre des particules comportant des traces d'acide

2

acétique, il vire au noir. Les gouttelettes n'ayant pas réagi se décolorent au bout de quelques minutes au contact de l'air.

Le troisième produit selon l'invention est destiné à la détection du cannabis. Il est constitué par deux réactifs qui doivent être employés l'un après l'autre, toujours par pulvérisation.

Le premier réactif est constitué par un mélange en proportions égales de :

Sodium hydroxyde

Ethanol

pouvant être dilué dans 1Ø fois son volume d'eau.

Le deuxième réactif est constitué par un mélange de

sel de Bleu solide (B.B. blue salt)

et de

Dichlorométhane

dans des proportions variant de Ø,5 à Ø,Ø5% pour le Sel de Bleu et 99,5% à 99,95% pour le Dichlorométhane.

Ces deux réactifs sont, comme dans les cas précédents, appliqués par pulvérisation.

Avec le premier réactif, qui est légèrement teinté en bleu, on recouvre toute la surface sur laquelle on pense qu'il y a des particules de cannabis présentes, puis immédiatement après, on procède à la pulvérisation du deuxième réactif. Par réaction avec le Tetra Hydro Cannabinol une coloration brun-orangé apparaît là où il y a des particules de cannabis.

## Revendications

1. Procédé de détection de particules de stupéfiants tels que cocaïne, héroïne et/ou cannabis, caractérisé par le fait qu'on pulvérise sur une surface susceptible de comporter des particules desdits stupéfiants, même en quantités infinitésimales, un réactif colorant liquide approprié au stupéfiant spécifique recherché.

2. Procédé selon la revendication 1, caractérisé par le fait qu'on frotte avec un papier absorbant blanc la surface soupçonnée d'avoir reçu des particules de stupéfiant et on pulvérise le réactif coloré sur ce papier.

3. Procédé selon les revendications 1 et 2 pour la détection de la présence, même en quantités infinitésimales, de particules de cocaïne, caractérisé par le fait que l'on emploie un réactif constitué par une solution dans l'eau d'un mélange de Chlorure de Cobalt et de Thiocyanate d'ammonium en tant que matières actives.

4. Procédé selon la revendication 3, dans lequel le mélange de matières actives est additionné d'une quantité égale en volume d'eau; de sorte que le produit comporte 5Ø % de matières actives et 5Ø % d'eau.

5. Procédé selon la revendication 3, dans lequel le mélange de matières actives est de 2 pour mille par rapport à l'eau.

6. Procédé selon les revendications 4 et 5, dans lequel les proportions respectives des deux matières actives peuvent varier de 1Ø % à 75 % pour le Chlorure de Cobalt et de 9Ø % à 25 % pour le Thiocyanate d'ammonium, la proportion préférentielle étant de 1/4 pour le Chrome de Cobalt et 3/4 pour le Thiocyanate d'ammonium.

7. Procédé selon la revendication 6, dans lequel les proportions préférentielles sont les suivantes :

Chlorure de Cobalt          1 %

Thiocyanate d'ammonium   3 %

Eau                               96 %

8. Procédé selon la revendication 7, caractérisé par le fait que le mélange est dilué dans 2Ø fois son volume d'eau.

9. Procédé selon l'une quelconque des revendications 4 à 8, dans lequel on peut remplacer partiellement l'eau par de l'Ethanol, jusqu'à environ 1Ø %.

10. Procédé selon les revendications 1 et 2 pour la détection de la présence, même en quantités infinitésimales, de particules d'héroïne, caractérisé par le fait que l'on emploie un réactif constitué par une solution dans l'eau de Bromophénol Bleu, dans une proportion allant de 1 % à 75 % pour le Bromophénol Bleu et donc de 99 % à 25 % pour l'eau; la proportion préférentielle étant de 4 % pour le Bromophénol Bleu et 96 % pour l'eau.

11. Procédé selon la revendication 1Ø, dans lequel on peut remplacer partiellement l'eau par de l'Ethanol, jusque dans la proportion de 2Ø %.

12. Procédé selon les revendications 1 et 2, pour la détection de la présence, même en quantités infinitésimales, de particules de cannabis, caractérisé par le fait que l'on emploie deux réactifs devant être employés l'un après l'autre, le premier réactif étant constitué d'une solution dans l'eau d'un mélange en proportions égales de Sodium Hydroxyde et d'Ethanol, le deuxième étant constitué par un mélange de Sel de Bleu solide et de Dichlorométhane.

13. Procédé selon la revendication 12, caractérisé par le fait que le mélange en proportions égales de Sodium Hydroxyde et d'Ethanol peut être dilué dans l'eau dans des proportions comprises entre 1 et 1∅ fois son volume d'eau.

14. Procédé selon la revendication 12, caractérisé par le fait que les proportions de Sel de Bleu solide et de Dichlorométhane sont comprises entre ∅,5 % et ∅,∅5 % pour le premier et 99,5 % et 99,95 % pour le second.

**Patentansprüche**

1. Verfahren zum Nachweis von Narkotika-Teilchen wie Kokain, Heroin und/oder Cannabis, dadurch gekennzeichnet, dass dass man auf eine Oberfläche, die geeignet ist, Teilchen der genannten Narkotika auch in infinitesimalen Mengen zu enthalten, ein flüssiges Färbe-Reagens aufsprüht, welches für das gesuchte, spezifische Narkotikum passend ist.

2. Verfahren nach Anspruch 1, dadurch gekennzeichnet, dass man mit einem weissen, absorbierenden Papier die Oberfläche, von der man vermutet, dass sie Teilchen des Narkotikums aufgenommen hat, abreibt, und dass man das Färbe-Reagens auf dieses Papier aufsprüht.

3. Verfahren nach den Ansprüchen 1 und 2 zum Nachweis des Vorliegens von Kokain-Teilchen auch in infinitesimalen Mengen, dadurch gekennzeichnet, dass man ein Reagens verwendet, das aus einer wässrigen Lösung eines Gemisches von Kobaltchlorid und Ammoniumthiocyanat als aktive Stoffe besteht.

4. Verfahren nach Anspruch 3, bei welchem dem Gemisch der aktiven Stoffe eine gleiche Volumenmenge Wasser zufügt wird, derart, dass das Produkt 5∅ % aktive Stoffe und 5∅ % Wasser enthält.

5. Verfahren nach Anspruch 3, bei welchem das Gemisch der aktiven Stoffe 2 Promille bezüglich des Wassers beträgt.

6. Verfahren nach den Ansprüchen 4 und 5, bei welchem die entsprechenden Proportionen der beiden aktiven Stoffe von 1∅ % bis 75 % für das Kobaltchlorid und von 9∅ % bis 25 % für das Ammoniumthiocyanat variieren können, wobei das bevorzugte Verhältnis 1/4 für das Kobaltchlorid und 3/4 für das Ammoniumthiocyanat beträgt.

7. Verfahren nach Anspruch 6, bei welchem die bevorzugten Verhältnisse die folgenden sind:

Kobaltchlorid          1 %
Ammoniumthiocyanat   3 %
Wasser                96 %

8. Verfahren nach Anspruch 7, dadurch gekennzeichnet, dass das Gemisch in der 2∅ fachen Menge seines Wasservolumens verdünnt wird.

9. Verfahren nach einem der Ansprüche 4 bis 8, bei welchem man das Wasser teilweise, bis zu etwa 1∅ %, durch Ethanol ersetzen kann.

1∅. Verfahren nach den Ansprüchen 1 und 2 zum Nachweis des Vorliegens von Heroin-Teilchen auch in infinitesimalen Mengen, dadurch gekennzeichnet, dass man ein Reagens verwendet, das aus einer wässrigen Lösung von Bromphenolblau in einem Verhältnis von 1 % bis 75 % Bromphenolblau und demnach von 99 % bis 25 % Wasser besteht, wobei das bevorzugte Verhältnis 4 % Bromphenolblau und 96 % Wasser beträgt.

11. Verfahren nach Anspruch 1∅, bei welchem man das Wasser teilweise, bis zu einem Verhältnis von 2∅ %, durch Ethanol ersetzen kann.

12. Verfahren nach den Ansprüchen 1 und 2 zum Nachweis des Vorliegens von Cannabis-Teilchen auch in infinitesimalen Mengen, dadurch gekennzeichnet, dass man zwei nacheinander anzuwendende Reagenzien verwendet, wobei das erste Reagens aus einer wässrigen Lösung eines Gemisches von Natriumhydroxid und Ethanol zu gleichen Teilen besteht, und das zweite Reagens aus einer Mischung von festem Blausalz (Sel de Bleu; B.B. blue salt) und Dichlormethan besteht.

13. Verfahren nach Anspruch 12, dadurch gekennzeichnet, dass das Gemisch aus gleichen Teilen von Natriumhydroxid und Ethanol in Wasser in Verhältnissen zwischen einmal und zehnmal seines Wasservolumens verdünnt werden kann.

14. Verfahren nach Anspruch 12, dadurch gekennzeichnet, dass die Verhältnisse des festen Blausalzes und des Dichlormethans zwischen ∅,5 % und ∅,∅5 % für das erstere und zwischen 99,5 % und 99,95 % für das zweite liegen.

**Claims**

1. A method for detecting particles of narcotics such as cocaine, heroin and/or cannabis, characterized by the step of spraying over a surface liable to include particles of the said narcotics, even in infinitesimal amounts, a liquid colouring reagent which is appropriated to the specific narcotic being searched for.

2. A method according to Claim 1, characterized by the steps of rubbing with a piece of white absorbent paper the surface which is suspected of having received particles of a narcotic and of spraying the colouring reagent onto this paper.

3. A method according to Claims 1 and 2 for detecting the presence, even in infinitesimal amounts, of particles of cocaine, characterized by the fact of using a reagent constituted by a solution in water of a mixture of cobalt chloride and ammonium thiocyanate, as active substances.

4. A method according to Claim 3, wherein there is added to the mixture of active substances an equal quantity of water, by volume, so that the product comprises 5Ø % active substances and 5Ø % water.

5. A method according to Claim 3, wherein the mixture of active substances is 2 parts per thousand, in relation to the water amount.

6. A method according to Claims 4 and 5, wherein the respective proportions of the two active substances may vary from 1Ø % to 75 % for the cobalt chloride, and from 9Ø % to 25 % for the ammonium thiocynanate, a preferred proportion being 1/4 for the cobalt chloride and 3/4 for the ammonium thiocyanate.

7. A method according to Claim 6, wherein the preferred proportions are the following :

cobalt chloride 1 %
ammonium thiocyanate 3 %
water 96 %

8. A method according to Claim 7, characterized in that the mixture is diluted in 2Ø times its volume of water.

9. A method according to any one of Claims 4 - 8 wherein water may be partly replaced with ethanol, up to approximately 1Ø%.

1Ø. A method according to Claims 1 and 2 for the detection of the presence, even in infinitesimal amounts, of particles of heroin, characterized by using a reagent consisting of an aqueous solution of blue bromophenol in a proportion ranging from 1 % to 75 % for the blue bromophenol, and therefore ranging from 99 % to 25 % for the water, a preferred proportion being 4% blue bromophenol with 96 % water.

11. A method according to Claim 1Ø, wherein water may be partly replaced with ethanol, up to a proportion of 2Ø %.

12. A method according to Claims 1 and 2, for detecting the presence, even in infinitesimal amounts, of particles of cannabis, characterized in that there are utilized two reagents which must be used, one after the other, the first reagent being formed of an aqueous solution of a mixture containing, in equal proportions, sodium hydroxide and ethanol, the second reagent being formed of a mixture of solid B.B. blue salt and dichloromethane.

13. A method according to Claim 12, characterized in that the mixture of sodium hydroxide and ethanol in equal proportions may be diluted in water within proportions comprised between 1 and 1Ø times its volume of water.

14. A method according to Claim 12, characterized in that the proportions of solid B.B. blue salt and dichloromethane are comprised between Ø.5 % and Ø.Ø5 % for the first one and 99.5 % and 99.95 % for the second one.